(19) Europäisches Patentamt
European Patent Office
Office européen des brevets

(11) **EP 2 264 461 A1**

(12) **EUROPÄISCHE PATENTANMELDUNG**

(43) Veröffentlichungstag:
**22.12.2010 Patentblatt 2010/51**

(51) Int Cl.:
*G01N 33/543* (2006.01)          *B01L 3/00* (2006.01)
*G01N 30/60* (2006.01)

(21) Anmeldenummer: **09163263.8**

(22) Anmeldetag: **19.06.2009**

(84) Benannte Vertragsstaaten:
**AT BE BG CH CY CZ DE DK EE ES FI FR GB GR
HR HU IE IS IT LI LT LU LV MC MK MT NL NO PL
PT RO SE SI SK TR**

(71) Anmelder:
• **FZMB GmbH Forschungszentrum für
Medizintechnik
und Biotechnologie
99947 Bad Langensalza (DE)**
• **Senova Gesellschaft für Biowissenschaft und
Technik MBH
Winzerlaer Str. 2
07745 Jena (DE)**

(72) Erfinder:
• **Miethe, Peter
06632 Schleberoda (DE)**
• **Söffing, Hans Hermann
99423 Weimar (DE)**

(74) Vertreter: **von Kreisler Selting Werner
Deichmannhaus am Dom
Bahnhofsvorplatz 1
50667 Köln (DE)**

(54) **Vorrichtung zur Durchführung eines affinitätschromatographischen Probentests**

(57)     Die Vorrichtung zur Durchführung eines affinitätschromatographischen Probentests ist versehen mit einem Probenanalysegefäß, das ein oberes Ende mit einer Probeneinlassöffnung und ein unteres Ende mit einer Auslassöffnung aufweist, wobei das Probenanalysegefäfi von der Probeneinlassöffnung bis zur Auslaßöffnung von einer zu analysierenden, flüssigen, vorzugsweise wässrigen Probe durchströmbar ist. Ferner ist das Probenanalysegefäß versehen mit einer sich an die Probeneinlassöffnung anschließenden Probenkammer zur Aufnahme einer Probe, einem stromab der Probenkammer angeordneten Markierungsmittelträger, der eine poröse Festphase und getrocknetes Marklerungsmittel aufweist, und mindestens einem stromab des Markierungsmitteiträgers angeordneten Affinitätsligandenträger, der eine poröse Festphase mit immobilisierten Affinitätsliganden aufweist. Der Strömungswiderstand des Markierungsmittelträgers ist im Verhältnis zum Strömungswiderstand der stromab des Markierungsmittelträgers befindlichen Anordnung derart, dass ein erstes Teilvolumen der Probe den Markierungsmittelträger schneller durchströmt als ein stromab des Markierungsmittelträgers benachbart zu diesem befindliches poröses Durchströmelement, bei dem es sich entweder um den Affinitätsligandenträger oder ein Abstandselement zur Beabstandung des Affinitätsligandenträgers von dem Markierungsmittelträger handelt.

Fig.1

**Beschreibung**

Die Erfindung betrifft eine Vorrichtung zur Durchführung eins affinitätschromatographischen Probentests sowie die Verwendung einer derartigen Vorrichtung.

Probentests (auch Assays genant) sind in den unterschiedlichsten Ausgestaltungen bekannt. Beispielsweise existieren Streifentests (siehe EP-B-0 306 772) als integriertes Einschrittdevice mit Funktionselementen Probenaufgabefeld, Konjugatreleasepad (Markierungsmittelträger mit freigebbaren Markierungsmittel bzw. -reaktanten) und Nitrozellulosemembran, die über Kapillarkräfte den Flüssigkeitstransport zu einem oder mehreren Reaktionsfeldern (Linien) gewährleistet, innerhalb derer sich im wesentlichen lediglich auf der Oberfläche des Streifenmaterials (auch Membran genannt) immobilisierte Fängerantikörper bzw. Affinitätsliganden befinden, sowie Abfallpad (saugfähiges Material) (Lateral-Flow-Test). Ein Teilvolumen der Probe reagiert in einer vorgelagerten Reaktion mit dem Markierungsmittel bzw. Konjugat, wandert über die Membran zu den Reaktionsfeldern und reagiert dort mit den Fängerantikörpern. Mit überschüssigem Probenvolumen wird die Membran "gewaschen" und die Flüssigkeit wird im Abfallpad aufgesaugt. Der Hauptvorteil ist die kompakte Bauweise und einfache Herstellung und Handhabung. Die Hauptnachteile sind schlechte Präzision und Reproduzierbarkeit der quantitativen Auswertung. Dies ist durch folgende Effekte verursacht:

i) aufgrund von Flussratenschwankungen, die durch Porositätsschwankung der Membran verursacht wird, schwankt die Verweilzeit der Probe in den einzelnen Funktionselementen;

ii) Nachreaktionseffekte, d.h. die Farbintensität der Banden ändert sich nach dem Durchströmen der Probe durch die Anordnung in einem Zeitfenster von 5 bis ca. 30 Minuten stark;

iii) außerdem kann im Produktionsprozess der Fall auftreten, dass die Fängerantikörper lediglich an der Membranoberfläche immobilisiert sind, so dass während eines Tests Probenflüssigkeit vollständig oder teilweise unter diesem Oberflächenimmobilisat durch die Membran strömt, ohne eine Reaktion auszulösen. Durch diesen "Tunneleffekt" können falsch negative Ergebnisse auftreten.

Aus z.B. EP-B-0 634 015, EP-B-0 557 288 und WO-A-95/19569 sind Durchfluss-Immunoassays auf Basis von dreidimensionalen Filtern, Gelen bzw. Schüttungen bekannt. Möglichkeiten zur optischen Direktauswertung auf der Säule sind in DE-10 2004 006 470, DE 103 09 132 und WO-A-2008/45722 beschrieben. Der Hauptnachteil der Test nach EP-B-0 634 015 ist die Notwendigkeit, die einzelnen Assayschritte, nämlich Zugabe der Probe, Zugabe des Konjugates, Waschlösung(en), Substratlösung manuell nacheinander aufzugeben (Mehrschrittassay).

Aufgabe der Erfindung ist es, ein Verfahren zur Durchführung eines affinitätschromatographischen Probentests und deren Verwendung anzugeben, wobei der Probentest einfach, reproduzierbar und zuverlässig abläuft und damit auch eine Quantifizierung mit guter Präzision zulässt, wobei die dabei eingesetzten Komponenten einfach herstellbar und die Vorrichtung einfach und kosteneffizient konfektionierbar sein soll.

Zur Lösung dieser Aufgabe wird mit der Erfindung eine Vorrichtung zur Durchführung eines affinitätschromatographischen Probentests vorgeschlagen, wobei die Vorrichtung versehen ist mit

- einem Probenanalysegefäß, das ein oberes Ende mit einer Probeneinlassöffnung und ein unteres Ende mit einer Auslassöffnung aufweist,
- wobei das Probenanalysegefäß von der Probeneinlassöffnung bis zur Auslassöffnung von einer zu analysierenden, flüssigen, vorzugsweise wässrigen Probe durchströmbar ist und
- wobei das Probenanalysegefäß versehen ist mit

  - einer sich an die Probeneinlassöffnung anschließenden Probenkammer zur Aufnahme einer Probe,
  - einem stromab der Probenkammer angeordneten Markierungsmittelträger, der eine poröse Festphase und Markierungsmittel sowie optional Vorreaktionsreagenzien (und zwar jeweils in getrockneter Form z.B. durch Lyaphilisierung) aufweist, und
  - mindestens einem stromab des Markierungsmittelträgers angeordneten Affinitätsligandenträger, der eine poröse Festphase mit immobilisierten Affinitätsliganden aufweist,
  - wobei der Strömungswiderstand des Markierungsmittelträgers im Verhältnis zum Strömungswiderstand der stromab des Markierungsmittelträgers befindlichen Anordnung derart ist, dass ein erstes Teilvolumen der Probe den Markierungsmittelträger schneller durchströmt als ein stromab des Markierungsmittelträgers benachbart zu diesem befindliches poröses Durchströmelement, bei dem es sich entweder um den Affinitätsligandenträger oder ein Abstandselement zur Beabstandung des Affinitätsligandenträgers von dem Markierungsmittelträger handelt.

Bei der erfindungsgemäßen Vorrichtung handelt es sich im Wesentlichen um ein Probenanalysegefäß von dessen in Gebrauchslage oberen Ende bis zu dessen in Gebrauchslage unteren Ende eine zu analysierende Probe strömt. Dabei wird neben der Kapillarkraft der porösen Elemente der hydrostatische Druck, der auf die zu analysierende flüssige, insbesondere wässrige Lösung einwirkt, als wesentliche treibende Kraft zum Durchströmen des Probenanalysegefäßes genutzt.

Innerhalb des Probenanalysegefäßes befindet sich zunächst eine sich an die Probeneinlassöffnung anschließende Probenkammer zur Aufnahme einer Probe. Die Probenkammer ist gegenüber dem verbleibenden Teil des Probenanalysegefäßes durch ein Markierungsmittelträger getrennt, der eine poröse Festphase mit Markierungsmittel sowie optional Vorreaktionsreagenzien aufweist, welches bzw. welche über eine Form der Trocknung, insbesondere durch Lyophilisierung, in den Markierungsmittelträger eingebracht ist/sind. Dieses Markierungsmittel (nachfolgend auch Konjugat bzw. Markierungsreaktant genannt) und die optionalen Vorreaktionsreagenzien vermischen sich mit einem ersten Teilvolumen der zu analysierenden Probe sehr schnell, wodurch eine Bindungsreaktion des Markierungsmittels oder und der Vorreaktionsreagenzien mit dem zu analysierenden Analyten der Probe initiiert wird.

Stromab des Markierungsmittelträgers befindet sich nächstbenachbart zu diesem ein poröses Durchströmelement, bei dem es sich z.B. um mindestens ein Affinitätsligandenträger handelt, der eine poröse Festphase mit immobilisierten Affinitätsliganden aufweist. Zwischen dem Markierungsmittelträger und dem Affinitätsligandenträger können zusätzlich ein oder mehrere poröses Abstandselemente als Durchströmelement angeordnet sein.

Der affinitätschromatographische Probentest nach der Erfindung, läuft nun beispielsweise im häufig eingesetzten Doppelantikörpersandwichformat dergestalt ab, dass an dem im Affinitätsligandenträger immobilisierten Fängerantikörper (Affinitätsligand) der Analyt der Probe, der in einer Vorreaktion einen Komplex mit einem Farbstoff-Antikörper Konjugat (Markierungsmittel) gebildet hat, bindet und somit im Affinitätsligandenträger verbleibt, während die Probe das Probenanalysegefäß bis zu dessen Auslassöffnung durchströmt.

In einem kompetitiven Assayformat wird das Markierungsmittel, das im Markierungsmittelträger mit der Probe vermischt wurde, selbst in Konkurrenz mit dem Analyten an den Liganden des Affinitätsligandenträgers gebunden. Die Menge an im Affinitätsligandenträger gebundenem Analyt kann in jedem Falle und in Abhängigkeit von der verwendeten Markierung des Markierungsmittels dann messtechnisch ermittelt werden, was beispielsweise optisch, magnetisch und/oder elektrochemisch erfolgen kann. Dies ist an sich im Stand der Technik bekannt.

Erfindungsgemäß ist nun der Strömungswiderstand des Markierungsmittelträgers im Verhältnis zum Strömungswiderstand der stromab des Markierungsmittelträgers befindlichen Anordnung (beispielsweise Affinitätsligandenträger mit oder ohne Abstandselement) derart gewählt, dass der Markierungsmitteiträger von einem ersten Teilvolumen der Probe schneller durchströmbar ist als die stromab des Markierungsmittelträgers befindliche Filteranordnung. Dadurch wird erreicht, dass das erste Teilvolumen der Probe das gesamte Porenvolumen des Markierungsmittelträgers vollständig ausfüllt, bevor das erste Teilvolumen der Probe in das stromab des Markierungsmittelträgers befindliche Element der Filteranordnung eintritt. Damit wird sichergestellt, dass sich die zu analysierende Probe in ausreichender und reproduzierbarer Weise mit dem Markierungsmittel und/oder mit den optionalen Vorreaktionsreagenzien mischt, wodurch eine Reaktion zur Komplexbildung des Analyten mit dem Markierungsreagenz und den Vorreaktionsreagenzien initiiert werden kann, so dass dann, wenn die Probe und das Markierungsmittel bzw. der Komplex aus Probe und Markierungsmittel bzw. aus Probe Markierungsmittel und Vorreaktionsreagenz den Affinitätsligandenträger durchströmt, es dort zu den gewünschten Bindungsreaktionen kommt, was für den einwandfreien Ablauf des Probentests unerlässlich ist. Dies gilt auch für kompetitive Assays, wobei es hier Assayverfahren gibt, bei denen es oberhalb des Affinitätsligandenträgers nur zur Vermischung der Probe mit dem Markierungsmittel bzw. den Vorreaktionsreagenzien kommt.

Die Strömungswiderstände der Festphasen von Markierungsmittelträger, Affinitätsligandenträger und ggf. vorhandenem Abstandselement lassen sich vor allem durch die Wahl der Porengrößen und/oder dem Porenvolumenanteil und/oder die Benetzungseigenschaften bzw. allgemein ausgedrückt durch die Grenzflächenspannung, d.h. die Wechselwirkungsenergie der Oberflächen der Festphasen zur Flüssigphase einstellen. Je nach Oberflächenbeschichtung der Festphasen und der Beschaffenheit der Probe (z.B. im wesentlichen wässrig) lässt sich die Grenzflächenspannung für die Festphase gezielt einstellen, um den Strömungswiderstand in einer gewünschten Art und Weise zu beeinflussen.

Zweckmäßigerweise weisen der Markierungsmittelträger, das Abstandselement und der Affinitätsligandenträger unterschiedliche poröse Festphasen auf. Bei dem Markierungsmittelträger handelt es sich vorzugsweise um ein Substrat aus Fasern, wobei die Fasern im wesentlichen in Längserstreckung gerichtet sind und die Festphase insbesondere einen Porenanteil von 20 % bis 80 % mit einem mittleren Porendurchmesser von 5 $\mu$m bis 200 $\mu$m aufweist und die Fasern Polyolefin, Polyester, Zellulose, Zellulosederivat oder Glas aufweisen, während der Affinitätsligandenträger (und ein ggf. vorhandenes poröses Abstandselement) typischerweise als gesintertes Substrat aus vorzugsweise Polymermaterial, wie z.B. Polyolefin, Teflon oder aus Glas vorliegt. Derartige Materialien können leicht zu Formstücken verarbeitet werden, die einfach in Rohre mit unterschiedlichen Querschnitten eingesteckt werden. Damit ist eine sehr einfache Konfektionierung möglich. Alternativ dazu können zu ihrer Herstellung aber auch Fasermaterialien mit Längsstreckung oder ohne Vorzugsrichtung der Fasern (Filze) verwendet werden.

Die Strömungswiderstände von Markierungsmittelträger und Affinitätsligandenträger bzw. Abstandselement wird zweckmäßigerweise mit dem sogenannten Wasserdurchlässigkeitsbeiwert umschrieben, der entsprechend der DIN 18130-1 "Bestimmung des Wasserdurchlässigkeitsbeiwerts" bestimmt wird.

Generell ist der Strömungswiderstand des Markierungsmittelträgers kleiner als der Strömungswiderstand des stromab des Markierungsmittelträgers befindlichen Durchströmelements (also kleiner als der Strömungswiderstand des Affinitätsligandenträgers bzw. eines eventuell vorhandenen porösen Abstandselements). Insbesondere sind die Strömungswiderstände durch die Durchlässigkeitsbeiwerte von Markierungsmittelträger und Affinitätsligandenträger bzw. Abstandselement definiert, wobei der Durchlässigkeitsbeiwert des Markierungsmittelträgers größer ist als der Durchlässigkeitsbeiwert des Affinitätsligandenträgers bzw. des porösen Abstandselements, Das stromab des Markierungsmittelträgers angeordnete Durchströmelement (Afifiinitätsligandenträger oder Abstandselement) führt also wegen seines gegenüber dem Markierungsmittelträger kleineren Durchlässigkeitsbeiwerts zu einer Verlangsamung der Durchströmrate der Probe durch die weitere Filteranordnung.

Der wesentliche Vorteil der erfindungsgemäßen Vorrichtung ist darin zu sehen, dass ein dreidimensionaler Markierungsmittelträger verwendet wird, der über seinen gesamten, von der Probe durchströmten Querschnitt hinweg mit Markierungsmittel versehen ist, wobei durch die Erhöhung des Strömungswiderstandes des stromab des Markierungsmittelträgers zu diesem nächstbenachbarten, insbesondere an diesen angrenzenden Durchströmelement gegenüber dem Markierungsmittelträger (bzw. durch die Verringerung des Strömungswiderstandes in den Markierungsmittelträger gegenüber dem Durchströmelement) erreicht wird, dass sich der Probendurchfluss nach dem Durchdringen des Markierungsmittelträgers verlangsamt und es zu einem vollständigen Ausfüllen des Porenvolumens des Markierungsmittelträgers durch die Probe kommt, was wiederum für die reproduzierbare Mischung/Reaktion von Probenanalyt und Markierungsmittel und optionalen Vorreaktionsreagenzien notwendig ist.

Der erfindungsgemäße Aufbau der Vorrichtung wurde mehrfach getestet, und zwar unter Verwendung von firittenfieirmigen Markierungsmittelträgern (auch Konjugat Release Filter genannt) und Affinitätsligandenträgern (auch Messfilter genannt) bzw. Abstandselementen (auch Trennfilter genannt). Der Durchlässigkeitsbeiwert des Markierungsmittelträgers betrug zwischen $3{,}0 \times 10^{-5}$ m/s bis $5{,}0 \times 10^{-5}$ m/s, vorzugsweise $3{,}0 \times 10^{-5}$ m/s bis $4{,}0 \times 10^{-5}$ m/s und insbesondere $3{,}2 \times 10^{-5}$ m/s bis $3{,}6 \times 10^{-5}$ m/s. Der Affinitätsligandenträger wies einen Durchlässigkeitsbeiwert zwischen $2{,}2 \times 10^{-5}$ m/s bis $3{,}2 \times 10^{-5}$ m/s, vorzugsweise $2{,}3 \times 10^{-5}$ m/s bis $2{,}5 \times 10^{-5}$ m/s auf. Schließlich betrug der Durchlässigkeitsbeiwert des Abstandselement zwischen $1{,}5 \times 10^{-5}$ m/s bis $2{,}0 \times 10^{-5}$ m/s, insbesondere $1{,}7 \times 10^{-5}$ m/s bis $1{,}9 \times 10^{-5}$ m/s.

Die Durchlässigkeitsbeiwerte von Markierungsmittelträger und Affinitätsligandenträger können auch untereinander vertauscht sein, d.h., dass die obigen Durchlässigkeitsbeiwertsbereiche des Markierungsmittelträgers für den Affinitätsligandenträger und die obigen Durchlässigkeitsbeiwertsbereiche des Affinitätsligandenträgers für den Markierungsmittelträger gelten.

Insgesamt hat es sich als zweckmäßig herausgestellt, wenn der Durchlässigkeitsbeiwert des Markierungsmittelträgers etwa das 1,5 bis 5-fache des Durchlässigkeitsbeiwerts des nächsten Durchströmelements (Affinitätsligandenträger oder Abstandselement) beträgt.

Der Affinitätsligandenträger kann einen Durchlässigkeitsbeiwert aufweisen, der etwa das 1,5 bis 2,5-fache des Durchlässigkeitsbeiwerts des ggf. vorhandenen Abstandselements aufweist.

Allgemein ausgedrückt kann das Abstandselement einen gegenüber dem Affinitätsligandenträger unterschiedlichen Strömungswiderstand aufweisen. Auf diesen Aspekt der Erfindung wird weiter unten noch eingegangen werden.

Durch die reproduzierbare Durchmischung bzw. Bindungsreaktion zwischen dem Analyten der Probe und dem Markierungsmittel bzw. den optionalen Vorreaktionsreagenzien im ersten Teilvolumen der Probe, dass den Markierungsmittelträger im wesentlichen vollständig ausfüllt, bevor dieses erste Teilvolumen den Rest der Anordnung des Probenaufnahmegefäßes durchströmt, wird gewährleistet, dass es bereits dann, wenn das erste Teilvolumen der Probe mit dem Markierungsmittel das Probengefäß durchströmt und in den bzw. die Affinitätsligandenträger eintritt, dort zu reproduzierbaren Bindungsreaktionen des in definierter reproduzierbarer Weise gebildeten Analyt-Markierungsmittel bzw. Analyt-Markierungsmittel- Vorreaktionsreagenzkomplex kommt, so dass anhand einer späteren Detektion dieser im Affinitätsligandenträger gebundene Analyt erfasst werden kann. Die Probenflüssigkeitssäule, die nach dem Befüllen des Markierungsmittelträgers noch in der Probenkammer verbleibt, kann nun in vorteilhafter Weise zum Waschen des bzw. der Affinitätsligandenträger genutzt werden. Dieser Waschschritt ist bei den bekannten Probentests mit säulenförmigem Testvorrichtungsaufbau als separater Schritt nach dem Durchströmen der Probe vorgesehen. Dieser separate Schritt kann nun erfindungsgemäß entfallen, indem die überschüssige in der Probenkammer zum Waschen und damit zum Herausspülen unspezifisch gebundener Analyten aus dem bzw. jedem Affinitätsligandenträger dient.

Das Volumen der in der Probenkammer nach Ausfüllen des Markierungsmittelträgers noch verbleibenden zweiten Teilvolumens in Relation zum Gesamtporenvolumen der Anordnung innerhalb des Probengefäßes ist entsprechend zu dimensionieren, wobei dieses zweite Teilvolumen zumindest gleichgroß wie das Gesamtporenvolumen der Anordnung ist. Zeckmäßigerweise entspricht das zweite Teilvolumen mindestens dem Gesamtporenvolumen. Besonders effektive Wascheffekte sind ab einem zweiten Teilvolumen, das 110%, vorzugsweise 120% des Gesamtporenvolumens entspricht, möglich.

Um mit der erfindungsgemäßen Vorrichtung affinitätschromatographische Probentests reproduzierbar durchführen zu können, ist es vorteilhaft, wenn die Durchflussraten der Probentestdurchführvorrichtungen im wesentlichen gleich sind, also nicht mehr als ein zulässiges Maß streuen. Durch Versuche hat sich überraschenderweise gezeigt, dass eine Vergleichsmäßigung der Durchströmraten erzielt wird, wenn eine Vorrichtung zum Einsatz kommt, die versehen ist mit

- einem Gefäß, das ein oberes Ende mit einer Probeneinlassöffnung und ein unteres Ende mit einer Auslassöffnung aufweist,
- wobei das Gefäß von der Probeneinlassöffnung bis zur Auslassöffnung von einer zu analysierenden, wässrigen Probe durchströmbar ist und
- wobei das Gefäß versehen ist mit

  - einer sich an die Probeneinlassöffnung anschließenden Probenkammer,
  - mehreren Affinitätsligandenträgern, die jeweils eine poröse Festphase mit immobilisierten Affinitätsliganden aufweisen, und
  - porösen Abstandselementen zur Beabstandung benachbarter Affinitätsligandenträger,

- wobei der Strömungswiderstand der Abstandselemente verschieden ist von dem Strömungswiderstand der Affinitätsligandenträger.

Zu diesem Teilaspekt der Erfindung wird hiermit auf die von den Anmelderinnen zeitgleich mit der vorliegenden Anmeldung eingereichte europäische Patentanmeldung mit dem Titel "Vorrichtung zur Affinitätsfiltration einer Flüssigkeit durch Mikrofilter" verwiesen, wobei der Inhalt dieser Anmeldung hiermit durch Bezugnahme zum Gegenstand der vorliegenden Anmeldung gemacht wird.

Die wechselweise Verwendung von bezüglich des Strömungswiderstandes unterschiedlicher Abstandselemente und Affinitätsligandenträger hat überraschenderweise gezeigt, dass sich die Strömungsraten gleicher Testanordnungen untereinander vergleichmäßigen.

Grundsätzlich umfasst der hierfür erforderliche Aufbau mindestens einen Affinitätsligandenträger, der stromauf und stromab von einem Abstandselement eingeschlossen ist. An dieser Anordnung können sich mehrere Paare aus Affinitätsligandenträger und Abstandselement anschließen. Besonders zweckmä-Big und im Sinne einer Vergleichsmäßigung der Durchströmraten vorteilhaft hat sich eine Anordnung herausgestellt, bei der drei Affinitätsligandenträger mit vier alternierend zu diesen angeordneten Abstandselementen vorgesehen sind. Entscheidend für die Vergleichmäßigung der Durchströmraten ist, dass Affinitätsligandenträger und Abstandselemente unterschiedliche Strömungswiderstände aufweisen, wobei es für die Erfindung insoweit irrelevant ist, ob nun das Abstandselement einen größeren Strömungswiderstand als der Affinitätsligandenträger oder der Affinitätsligandenträger einen größeren strömungswiderstand als das Abstandselement hat.

Auch für die Strömungswiderstände gilt wiederum, dass sie sich zweckmäßigerweise durch die materialspezifischen Durchlässigkeitsbeiwerte umschreiben lassen, die sich wiederum entsprechend der DIN 18130-1 "Bestimmung des Wasserdurchlässigkeitsbeiwerts" gemäß Versuchsklasse 3 bestimmen lassen. Als Fritten- bzw. Filtermaterial wurde ein übliches Frittenmaterial (nachfolgend Material 112 genannt) gewählt, das eine Porengröße von 10 $\mu$m aufwies (Durchschnittswert). Dieses Material 112 wurde für die Abstandselemente verwendet. Als Affinitätsligandenträger wurde ein Material mit einer durchschnittlichen Porengröße von 40 $\mu$m (nachfolgend mit Material 180 bezeichnet) verwendet. Schließlich wurden auch Untersuchungen mit einem weiteren Material durchgeführt, dessen Porengröße im Durchschnitt 60 $\mu$m betrug (nachfolgend mit Material 187 bezeichnet). Das Material 112 hatte einen Durchlässigkeitsbeiwert von 1,8 x $10^{-5}$ m/s, das Material 180 einen Durchlässigkeitsbeiwert von 2,6 x $10^{-5}$ m/s und das Material 187 einen Durchlässigkeitsbeiwert von 3,1 x $10^{-5}$ m/s. Diese Materialien lassen sich in Kombination miteinander einsetzen, wobei die wechselweise Anordnung zweier dieser Materialien den überraschenden Effekt hat, dass die Schwankungsbreite der Durchflussraten deutlich reduziert ist. Ferner wurden als Affinitätsligandenträger scheiben- bzw. zylinderförmige Messfritten aus dem Material 180 in den Dimensionen 2,5 mm (Höhe) x 5 mm (Durchmesser) (nachfolgend Typ1-Fritte genannt) sowie scheiben- bzw. zylinderförmige Abstandselemente aus dem Material 112 in den Dimensionen 1,6 mm (Höhe) x 5 mm (Durchmesser) (nachfolgend Typ3-Fritte genannt) getestet.

Der Durchlässigkeitsbeiwert verschiedener poröser Sinter- und Faserelemente wurde in enger Anlehnung an DIN 18130-1 (Versuchsklasse 3) bestimmt. In einem Standrohr (innerer Durchmesser 5 mm) wurden zwei Füllstandsmarkierungen h1 und h2 sowie eine Positionsmarkierung zur Einbringung des Filterelements angebracht. Eine konstante Flüssigkeitsmenge wurde in den oberen Einlass des Standrohres gegeben. Die Zeit, die der obere Flüssigkeitspegel von Markierung h1 bis Markierung h2 benötigte, wurde der Durchlässigkeitsbeiwertsmessung zu Grunde gelegt, wobei der Vorgang wiederholt wurde, bis sich eine konstante Durchflusszeit eingestellt hatte. Der Durchlässigkeitsbeiwert kf wurde anhand der nachfolgend genannten Formel für Filterelemente von je 5,0 mm Durchmesser und 1,6 mm Dicke bestimmt:

$$kf = \frac{1}{t} \times \ln\frac{h1}{h2}$$

In vorteilhafter Weiterbildung der Erfindung ist ferner vorgesehen, dass aus dem Probenanalysegefäß auslaufende Probenflüssigkeit von einem Abfallbehältnis aufnehmbar ist. Alternativ hierzu kann die Auslassöffnung des Probenaufnahmegefäßes mit einem Verschlusselement versehen sein, das sich (erst) bei Benetzung mit Flüssigkeit selbständig verschließt. Dadurch wird ein Austreten von Probenflüssigkeit aus dem Probenanalysegefäß verhindert, weshalb ein Abfallbehältnis entbehrlich ist.

Wie bereits oben erwähnt, lässt sich das Markierungsmittel (in dem Affinitätsligandenträger) optisch, nämlich beispielsweise photometrisch, fluormetrisch, luminometrisch, durch Messung der Phosphoreszenz oder magnetisch oder elektrochemisch, nämlich beispielsweise amperometrisch, potentiometrisch, konduktionmetrisch detektieren. Das Markierungsmittel lässt sich z.B. durch Konjugation eines Proteins, insbesondere eines Antikörpers, einer Nukleinsäure, eines Oligopeptids, oder eines Haptens mit einem oder mehreren Markermolekülen oder Partikeln herstellen.

Als Affinitätsligandenträger kommen sämtliche bei den bekannten Assays eingesetzte Bindungsmoleküle in Frage. Insbesondere eignen sich als Affinitätsliganden Proteine wie Antikörper, Protein A, Protein G, Streptavidin, native oder rekombinante Oberflächenantigene von Mikroorganismen, somatischen Zellen, oder Viren, Lektine oder Nukleinsäuren wie einsträngige RNA, DNA Sonden, Aptamere oder oligomeren Verbindungen wie Peptide, Oligosacharide oder niedermolekularen Verbindungen insbesondere Haptene oder Polysacharide insbesondere Zellulose.

Wie weiter oben bereits beschrieben, eignet sich die erfindungsgemäße Vorrichtung insbesondere auch dazu, den an sich bei Assays erforderliche Waschschritt mittels aus der Probenkammer nachlaufende Probenflüssigkeit zu realisieren. Von der Erfindung umfasst ist also auch die Verwendung der zuvor genannten Vorrichtung, wobei die Menge an zu analysierender, wässriger Probe derart gewählt wird, dass Markierungsmittel und/oder Markierungsmittel-Analyt-Komplexe, das/die in dem bzw. mindestens einem der Affinitätsligandenträger unspezifisch gebunden ist/sind, von nachlaufender Probe aus dem betreffenden Affinitätsligandenträger herausgewaschen wird/werden.

Die Erfindung wird nachfolgend anhand zweier Ausführungsbeispiele und unter Bezugnahme auf die Zeichnung näher erläutert. Im Einzelnen zeigen:

Fign. 1 bis 6    die verschiedenen Phasen während des Durchlaufens einer zu analysierenden Probe durch ein Probenaufnahmegefäß mit einer Fritenanordnung gemäß einem ersten Ausführungsbeispiel der Erfin- dung,

Fign. 7 bis 12    die verschiedenen Phasen während des Durchlaufens einer zu analy- sierenden Probe durch ein Probenaufnahmegefäß mit einer Frittenan- ordnung gemäß einem zweiten Ausführungsbeispiel der Erfindung,

Fig. 13    die Kalibrationskurve für die Vorrichtung gemäß Fign. 1 bis 6 und

Fig. 14    die Kalibrationskurve für die Vorrichtung gemäß Fign. 7 bis 12.

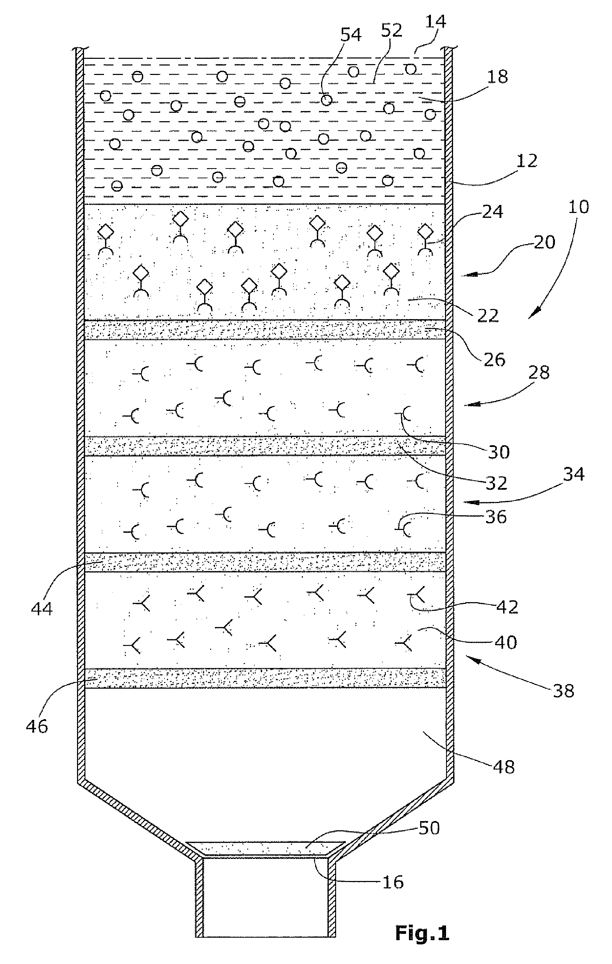

Fig. 1 zeigt den grundsätzlichen Aufbau einer Vorrichtung 10 zur Durchführung eines affinitätschromatographischen Probentests. Die Vorrichtung 10 umfasst ein Probenanalysegefäß 12, das am oberen Ende eine Einlassöffnung 14 und am unteren Ende eine Auslassöffnung 16 aufweist. An die Einlassöffnung 14 schließt sich eine Probenaufnahmekammer 18 an, die zur Auslassöffnung hin durch eine Anordnung diverser poröser Fritten bzw. Filter getrennt ist. Dieser Aufbau umfasst einen stromab der Probenaufnahmekammer 18 angeordneten Markierungsmittelträger 20, bei dem es sich um eine Konjugat Release Fritte handelt. Der Markierungsmittelträger 20 weist eine poröse Festphase 22 auf, in der sich Konjugat bzw. Markierungsmittel 24 befindet.

Stromab des Markierungsmittelträgers 20 befindet sich ein poröses Abstandselement 26 als Trennfritte. Auf das Abstandselement 26 folgt stromab ein poröser Affinitätsligandenträger 28 mit immobilisierten Affinitätsliganden 30, wobei dieser Affinitätsligandenträger 28 in diesem Ausführungsbeispiel über ein poröses Abstandselement 32 gegenüber einem weiteren Affinitätsligandenträger 34 mit Affinitätsliganden 36 getrennt ist. Die beiden Affinitätsligandenträger 28 und 34 bilden die Messfritten. Den Abschluss der Frittenanordnung bildet eine Kontrollfritte 38, die ein poröses Substrat 40 mit ebenfalls immobilisierten Affinitätsliganden 42 aufweist. Die Kontrollfritte 38 ist wiederum durch ein poröses Abstandselement 44 von dem Affinitätsligandenträger 34 getrennt und ist selbst durch ein poröses Abstandselement 46 gegenüber einem Restprobenauffangraum 48 oberhalb der Auslassöffnung 16 getrennt. In der Auslassöffnung 16 befindet sich ein Verschlusselement 50, das sich bei Kontakt mit Flüssigkeit automatisch verschließt. In diesem Falle schiebt die austretende Flüssigkeit die vor ihr liegende Luft durch die Röhrenanordnung, bis diese durch das Verschlusselement entweicht. Beispielsweise weist das Verschlusselement 50 Poren auf, die sich bei Kontakt des Verschlusselements 50 mit Wasser verschließen.

In der Probenaufnahmekammer 18 wird nun zu analysierende Probenflüssigkeit 52 mit einem Analyt 54 eingegeben.

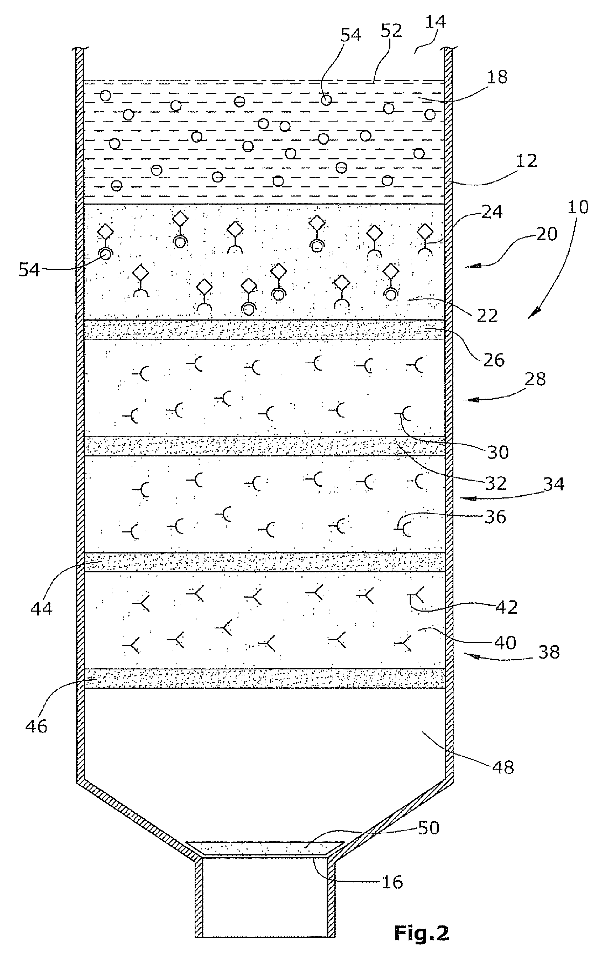

In einer ersten Phase des Tests gelangt nun ein erstes Teilvolumen der Probenflüssigkeit 52 in den Markierungsmittelträger 20, wobei die Probenflüssigkeit die Poren dieses Markierungsmittelträgers ausfüllt. In dem Markierungsmittelträger kommt es zu einer Bindungsreaktion zwischen dem Analyten 54 und dem Markierungsmittel 24, wie es in Fig. 2 gezeigt ist.

Durch Wahl der Strömungswiderstände bzw. Durchlässigkeitsbeiwerte der Materialien von Markierungsmittelträger 20 und Abstandselement 26 kann erreicht werden, dass der Flüssigkeitsstrom der Probe 52 nach dem Befüllen des Markierungsmittelträgers 20 verzögert wird und damit Probenflüssigkeit 52 mit Analyt 54 und Markierungsmittel 24 durch das Abstandselement 26 hindurch in den ersten Affinitätsligandenträger 28 verzögert eindringt (siehe Fig. 2). Im Affinitätsligandenträger 28 kommt es dann zu Bindungsreaktionen zwischen dem Analyt 54 und den Affinitätsliganden 30.

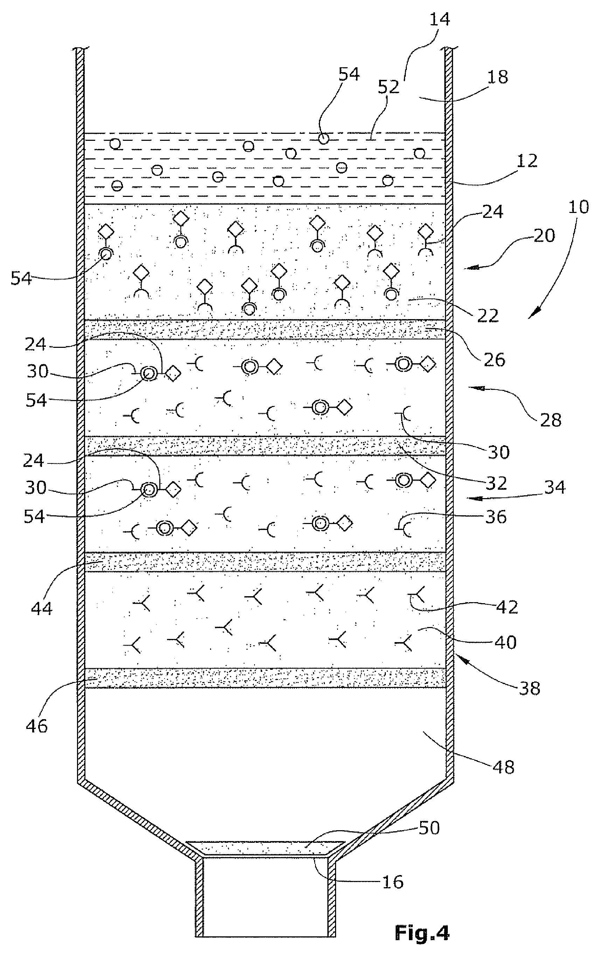

Mit weiterem Fortschreiten der Probenflüssigkeitsfront gelangt nun Probenflüssigkeit in den zweiten Affinitätsligandenträger 34, wo wiederum Analyt 54 an den Affinitätsliganden 36 bindet (seihe Fig. 4).

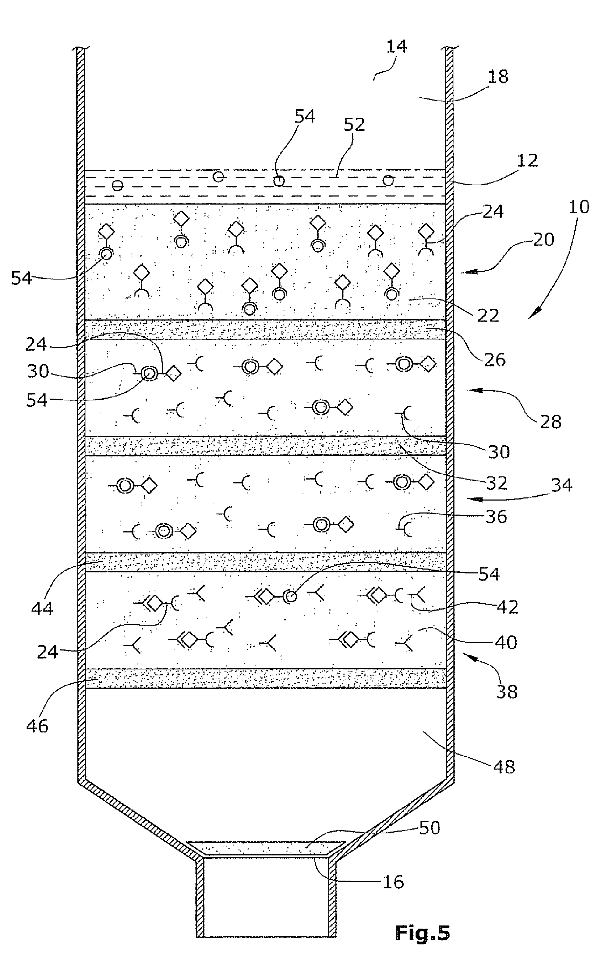

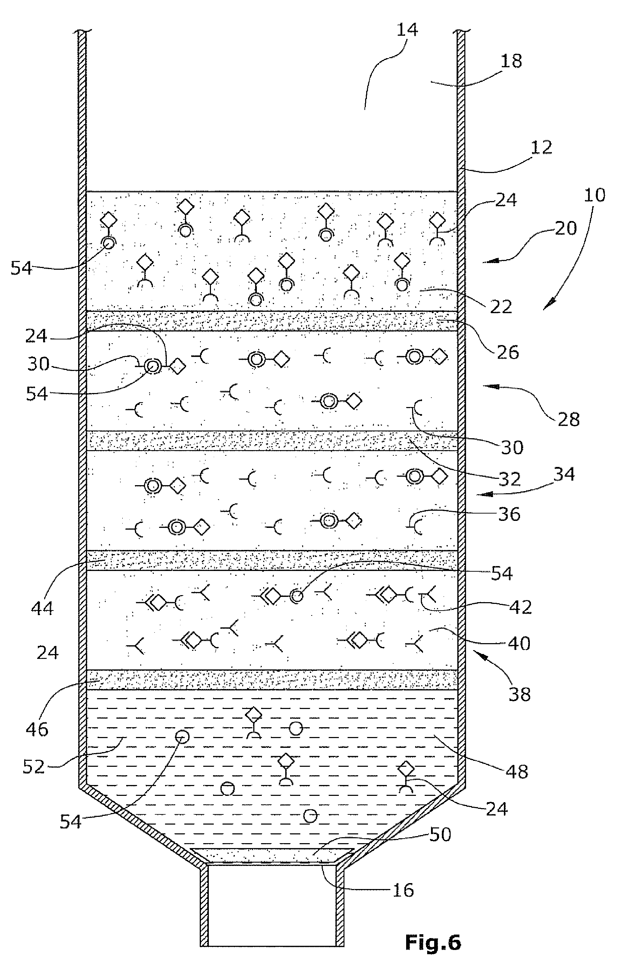

Des weiteren gelangt in einer nächsten Phase der Durchströmung der Probenflüssigkeit durch das Probenaufnahmegefäß 12 Markierungsmittel 24 ggf. mit daran gebundenem Analyt 54 in die Kontrollfritte 38, wo diese Substanzen bzw. Reaktanten an den Affinitätsliganden 42 binden (siehe Fig. 5). Anschließend gelangt die Probenflüssigkeit in den Restprobenauffangraum 48 (siehe Fig. 6). In der Folgezeit schiebt nun das überschüssige Teilvolumen der Probe in der Probenkammer 18 das erste Teilvolumen durch den Frittenaufbau vor sich her, wobei das zweite Teilvolumen der Probe zum Waschen, d.h. zum Herausspülen unspezifisch gebundener Analyt-Markierungsmittel-Komplexe genutzt wird. Hierdurch kann der Test als Einschritttest relativ schnell ablaufen.

Der Markierungsmittelträger 20 ist aus Fasern aufgebaut und weist einen geringeren Strömungswiderstand, d.h. einen größeren Durchlässigkeitsbeiwert als das Abstandselement 26 bzw. zumindest als der Affinitätsligandenträger 28 auf. Durch diese Abstimmung der Strömungswiderstände bzw. Durchlässigkeitsbeiwerte kommt es zu einer deutlichen Verlangsamung des weiteren Voranströmens der Probe, wodurch diese den Markierungsmittelträger 20 im wesentlichen vollständig durchflossen bzw. durchströmt hat, bevor sie in den ersten Affinitätsligandenträger 28 eintritt.

Durch die Verlangsamung der Strömung ist also der Markierungsmittelträger 20 im wesentlichen vollständig von dem ersten Teilvolumen der Probenflüssigkeit 52 "durchsetzt", so dass es zu einem intensiven Kontakt mit dem Markierungsmittel kommt (sei es durch ein Vermischen von Analyt und Markierungsmittel oder eine Reaktion beider Substanzen).

In der Folge entsteht dann durch die alternierende Anordnung der Affinitätsligandenträger 28,34 und der Kontrollfritte 38 einerseits und der Abstandselemente 26 andererseits eine über mehrere der Vorrichtungen 10 betrachtet gleichmäßigen Durchströmung. Dies wird erreicht, indem für die zuvor genannten alternierend angeordneten Elemente Materialien mit unterschiedlichen Porositäten bzw. Strömungswiderständen eingesetzt werden. Die Elemente mit den jeweils größeren Strömungswiderständen sorgen dabei stets für eine Verlangsamung des Fortschreitens der Flüssigkeitsfront und damit für eine Vergleichmäßigung der Strömungsraten. Hierdurch können die Tests reproduzierbar durchgeführt werden, da die Schwankungsbreiten der Verweildauern, für die sich die vorströmende Probenflüssigkeit in den Affinitätsligandenträgern befindet, vergleichmäßigt sind.

Die Erfindung wurde vorstehend anhand eines Ausführungsbeispiels beschrieben, bei dem der Analyt im Markierungsmittelträger zunächst an einem Markierungsmittel bindet, woraufhin dann der so entstehende Bindungskomplex wiederum an einem Liganden bindet. Die Erfindung ist aber auch einsetzbar bei sogenannten kompetitiven Assays, bei denen die Analyten und Markierungsmittel, ohne aneinander gebunden zu sein, die Affinitätsligandenträger durchströmen. Die Anordnung ist darüber hinaus auch für andere bekannte Bindungsassays einsetzbar.

Nachfolgend sowie vorausgehend sind verschiedene, im Rahmen von Versuchen verwendet scheiben- bzw. zylinderförmige Fritten mit Typ1, Typ2 und Typ3 bezeichnet, wobei Typ1 das Material 180 in 2,5 mm Höhe und 5 mm Durchmesser, Typ2 das Material 180 in 1,6 mm Höhe und 5 mm Durchmesser und Typ3 das Material 112 in 1,6 mm Höhe und 5 mm Durchmesser bezeichnet.

<u>BEISPIELE:</u>

Als Affinitätsligandenträger wurden Sinterkörper bestehend aus HDPE mit einem mittleren Porendurchmesser von 40 $\mu$m und einem Porenvolumenanteil von 50% in den Dimensionen (Durchmesser x Länge) 5x2,5 mm und einem Durchlässigkeitsbeiwert von 2,6 x $10^{-5}$ m/s (Typ1), eingesetzt auf denen 7,5 $\mu$g monoklonaler anti Hämoglobin Antikörper immobilisiert waren (Typ1 antiHäm =Typ1Hä)

Ebenso wurden Sinterkörper (Typ 1), auf welchen 7,5 $\mu$g anti Maus Antikörper (Typ1 Positivkontrolle = Typ1PK) immobilisiert waren, sowie ein mit Rinderserumalbumin beschichteter Sinterkörper (Typ 1) ohne spezifische Bindungsaktivität (Typ1 Negativkontrolle = Typ1NK) eingesetzt.

Des Weiteren wurden als Markierungsmittelträger ein zylinderförmiger Faserkörper in den Dimensionen 5 mm (Höhe) x 5 mm (Durchmesser) mit einem Durchlässigkeitsbeiwert von 4,1 x $10^{-5}$ m/s (MMT) eingesetzt. Auf diesen wurde im Herstellungsprozess 50 $\mu$l einer antiHämoglobin-Partikel-Konjugat-Suspension aufgebracht. In diesem Zustand wurde der Markierungsmittelträger getrocknet, und zwar lyophilisiert. Pro Markierungsmittelträger wurden so in trockener Form 125 $\mu$g Farbpartikel (Duke Scientific, D = 0,39 $\mu$m) konjugiert mit 4 $\mu$g antiHämoglobin Antikörper in trockener Form vorgelegt.

Als poröse Trenn- bzw. Abstandselemente wurden HDPE gesinterte Filterelemente in den Dimensionen 1,6 mm (Höhe) x 5 mm (Durchmesser) mit einem Durchlässigkeitsbeiwert von 1,8 x $10^{-5}$ m/s (Typ 3) ohne Bindungsaktivität (Blockfilter) eingesetzt.

Durch Einstecken von Filterelementen der zuvor definierten Typen 1, 3 in Mikrosäulen (Innendurchmesser Di = 5 mm, V = 750 μl) wurde eine Anordnung hergestellt, die der erfindungsgemäß vorgeschlagenen mehrlagigen Anordnung entspricht und durch sequenzielles Einstecken dem folgenden Schema entsprachen:

Filter Typ3/ Typ1PK/ Typ3/ Typ1Hä/ Typ3/ Typ1Hä/ Typ3/ MMT.

Als Kontrollmaterial wurde ein Hämoglobin-Standard verwendet. Die Konzentrationsangabe erfolgte auf Basis der auf den Beipackzetteln angegebenen Konzentration. Die Assays wurden in Pipettierständern mit eine Nullkontrolle und jeweils 6 Kalibrationsproben unterschiedlicher Konzentration in Doppelbestimmung durchgeführt. Die Zugabe der Probe erfolgte direkt in den Einlauf der Mikrosäule. Die Flussraten lagen dabei im Bereich von 500 μl in 6min. Nach Durchlauf der Probe (500 μl) wurde die Verfärbung der Sinterkörper (Typ 1) durch Transmissionsmessung quantifiziert. Das Assayschema stellt sich wie folgt dar:

- 500 μl Probe in Puffer
- Durchlauf in 6 min
- Bestimmung der Transmission

Das optische Auslesen der Referenzsäulen erfolgte mit einem Hand-Photometer, mit dem Transmissionsmessungen an dreilagigen Säulen bei einer Wellenlänge von 525nm realisiert werden konnten.

Aus den gemessenen Transmissionen wurde auf Basis der Formel OD = Log(I0/I) (mit I0 = Transmission einer ungefärbten Referenzsäule und I = Transmission des Messfilters) die optischen Dichten der einzelnen Filterelemente ermittelt. Zur Auswertung wurden die OD Werte der beiden Filter addiert. Diese korrelieren mit der Probenkonzentration, wobei eine sigmoide 4-Parameter-Logistik Gleichung zur Kalibration verwendet werden kann. Die Positivkontrollfelder zeigten in dem Experiment eine konstante optische Dichte OD im Bereich von 0,95 bis 1,07.

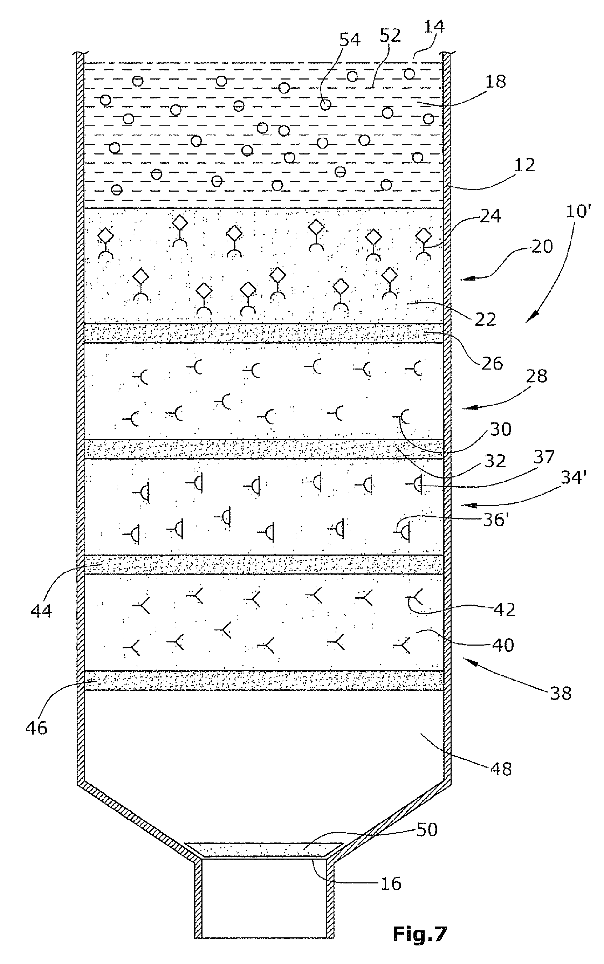

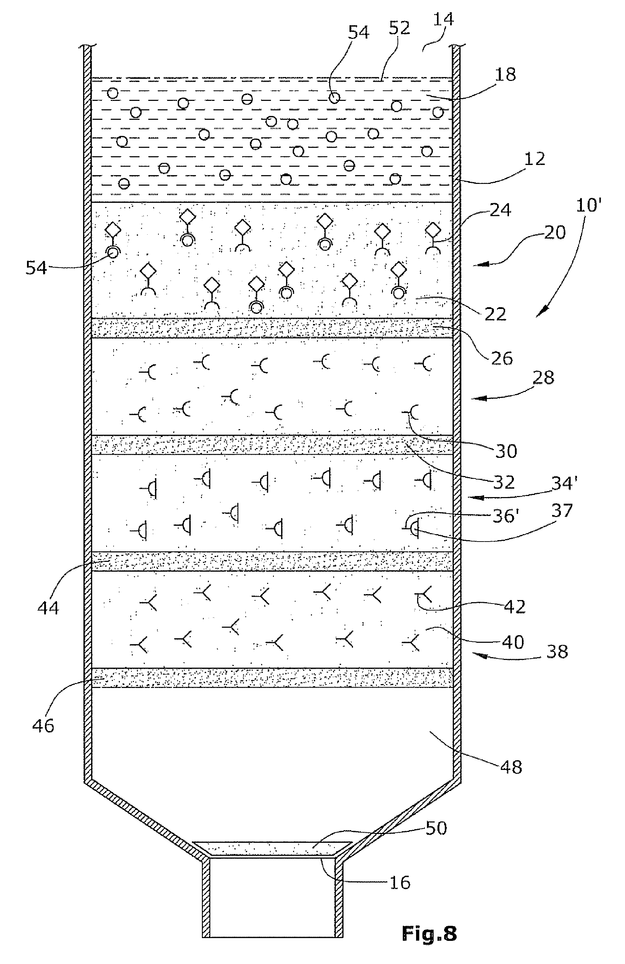

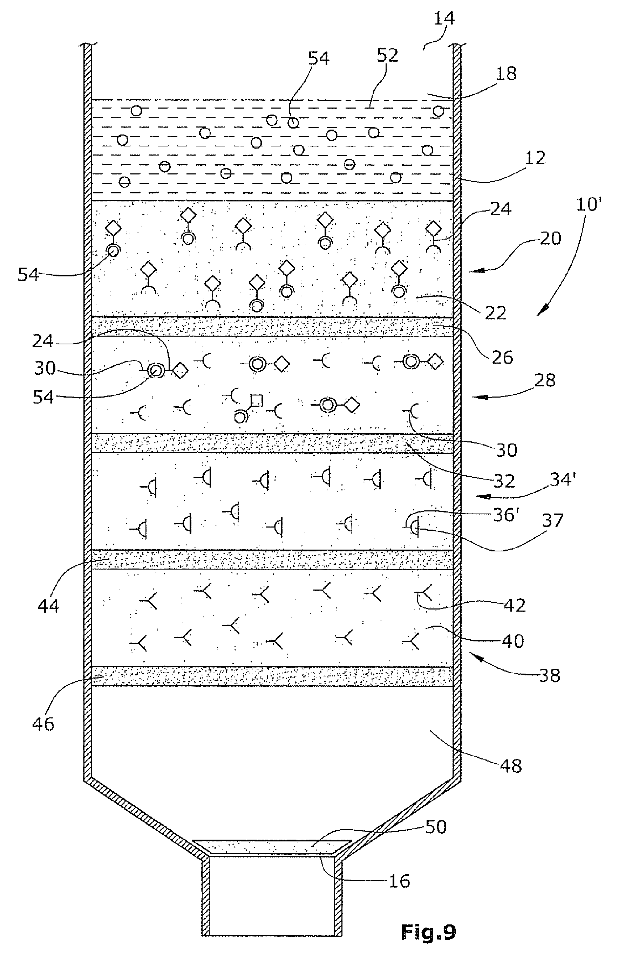

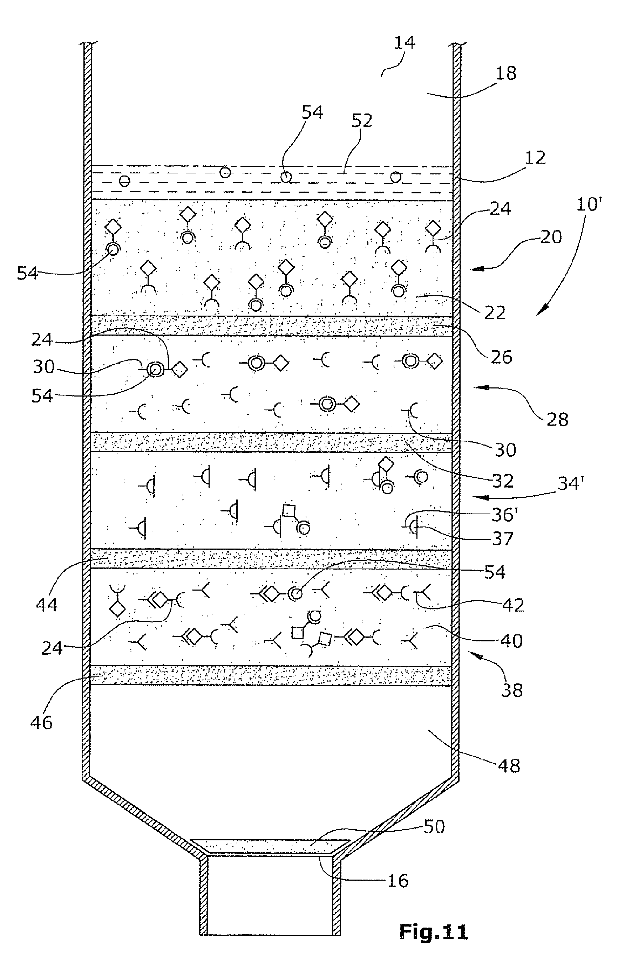

In den Fign. 7 bis 12 ist ein zweites Ausführungsbeispiel der Erfindung gezeigt. Es basiert auf der Anordnung:

Typ3/ Typ1PK/ Typ3/ Typ1NK/ Typ3/ Typ1Hä/ Typ3/ MMT

Beim zweiten Ausführungsbeispiel ist der erste Affinitätsligandenträger 28 als einzige Messfritte ausgebildet, an der der Analyt-Konjugat-Komplex bindet. Die im ersten Ausführungsbeispiel zweite Messfritte 34 ist im zweiten Ausführungsbeispiel als Negativ-Kontroll-Fritte 34' ausgebildet und umfasst immobilisierte Blockproteine 37. In der Negativ-Kontroll-Fritte dürfen nach Abschluss des Tests keine irgendwie geartete Bindungskomplexe sondern ausschließlich die Blockproteinen vorhanden sein.

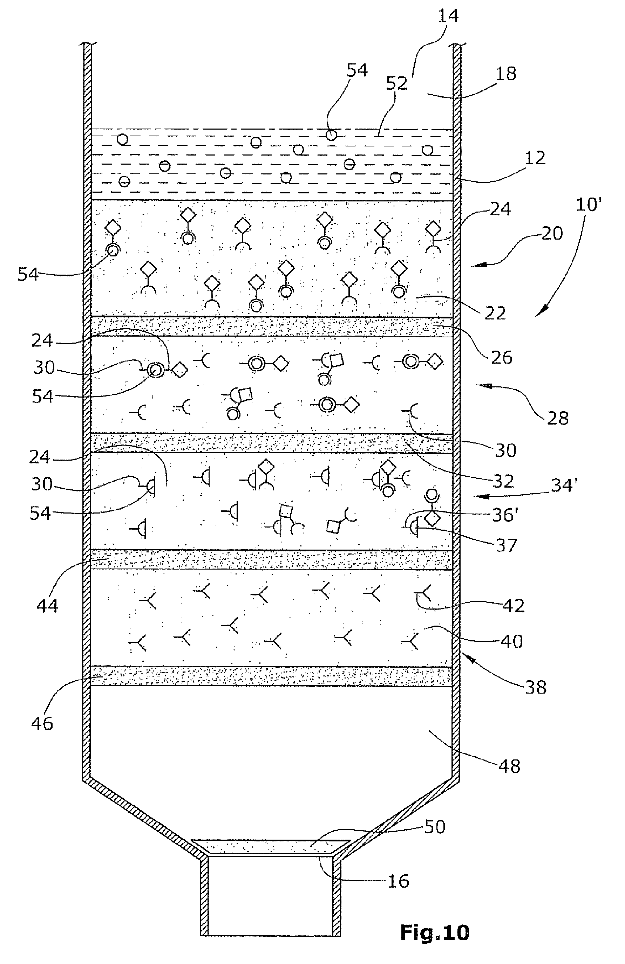

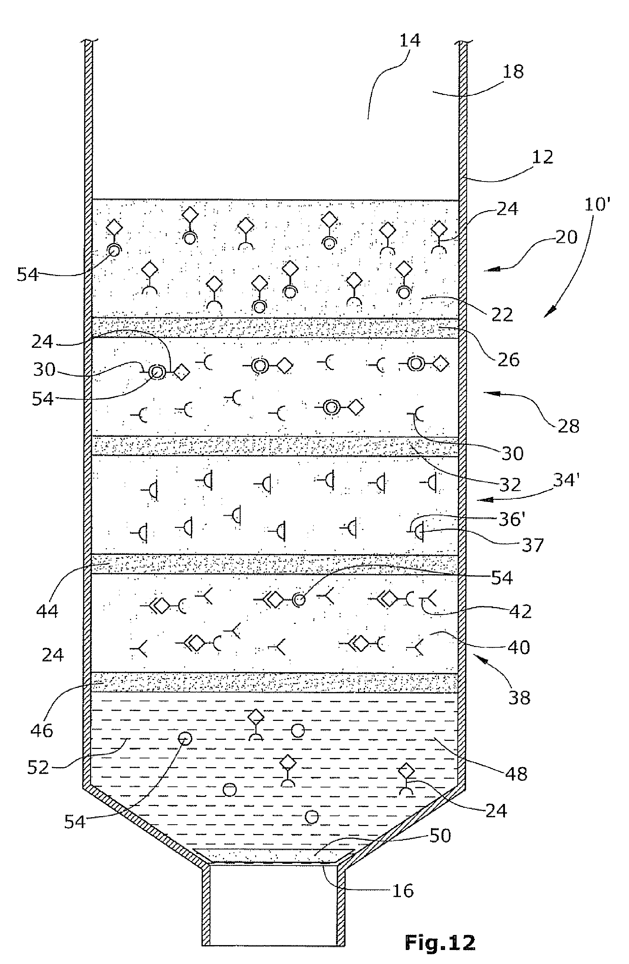

Wie man anhand von Fig. 10 erkennen kann, können nun Analyt-Konjugat-Komplexe unspezifisch an den Blockproteinen 37 oder Matrix der Negativ-Kontroll-Fritte 34 binden. Nach der Waschphase (siehe Fig. 12) sind diese Komplexe in der Negativ-Kontroll-Fritte 34' nicht mehr enthalten. Auch auf andere Weise in der Messfritte 28 sowie in der Negativ-Kontroll-Fritte 34' "festgehaltene" Komplexe sind nach dem Waschvorgang nicht mehr enthalten, womit sich die Situation gemäß Fig. 12 einstellt.

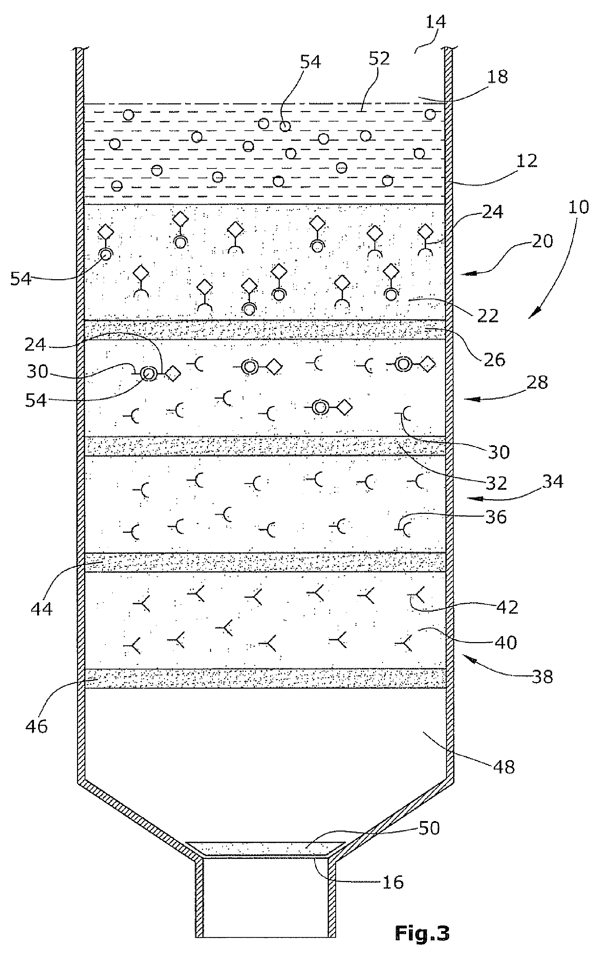

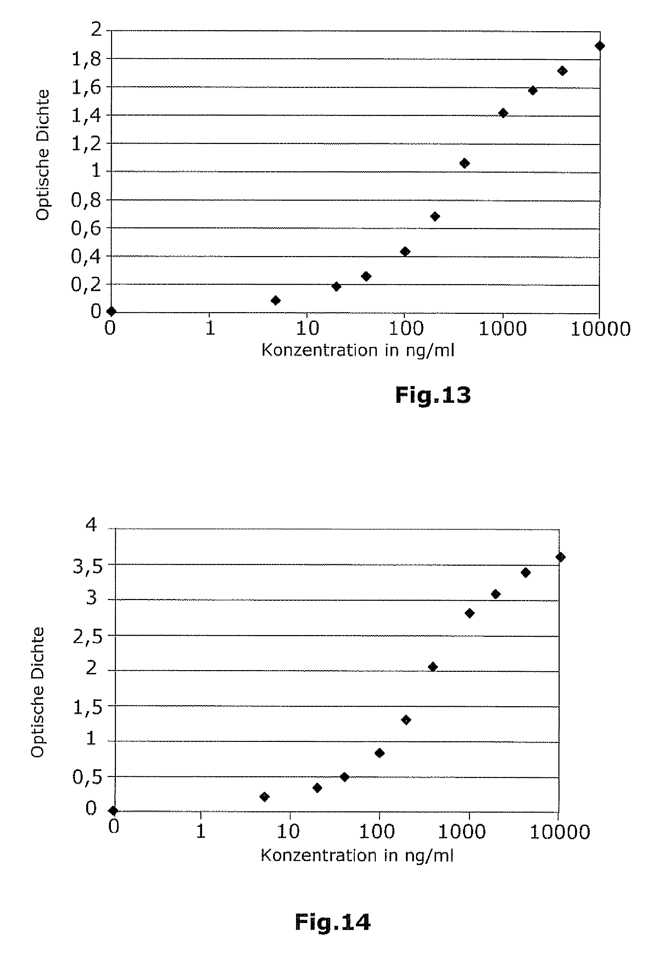

Der zuvor genannte Assay wurde unter Verwendung der Vorrichtungen gemäß den Fign. 1 bis 6 (Variante 1) und Fign. 7 bis 12 (Variante 2) für verschiedene Probenflüssigkeiten mit jeweils definierten Hämoglolbinkonzentrationen durchgeführt, wobei sich Kalibrationskurven gemäß Fig. 13 (für Variante 1) und Fig. 14 (für Variante 2) erstellen ließen. Auf den Y-Achsen ist jeweils die optische Dichte (OD als dimensionslose Größe) aufgetragen, während auf den X-Achsen die den gemessenen optischen Dichten entsprechenden Hämoglobinmengen in ng/ml logarithmisch aufgetragen sind.

Die Variationskoeffizienten (in %) an der unteren Nachweisgrenze (in diesem Fall 40 ng/ml - low VK genannt) an der oben Nachweisgrenze (in diesem Fall 2.000 ng/ml - high VK genannt) und in einem mittleren Nachweisbereich (in diesem Fall 400 ng/ml - medium VK genannt) sind in der nachfolgenden Tabelle angegeben.

| VK | Variante 1 | Variante 2 |
|--------|------------|------------|
| low | 7,9 | 8,4 |
| medium | 4,2 | 3,9 |
| high | 4,9 | 5,1 |

**Patentansprüche**

1. Vorrichtung zur Durchführung eines affinitätschromatographischen Probentests, mit

- einem Probenanalysegefäß, das ein oberes Ende mit einer Probeneinlassöffnung und ein unteres Ende mit einer Auslassöffnung aufweist,
- wobei das Probenanalysegefäß von der Probeneinlassöffnung bis zur Auslassöffnung von einer zu analysierenden, flüssigen, vorzugsweise wässrigen Probe durchströmbar ist und
- wobei das Probenanalysegefäß versehen ist mit

- einer sich an die Probeneinlassöffnung anschließenden Probenkammer zur Aufnahme einer Probe,

- einem stromab der Probenkammer angeordneten Markierungsmittelträger, der eine poröse Festphase und Markierungsmittel in getrocknetem Zustand aufweist, und
- mindestens einem stromab des Markierungsmittelträgers angeordneten Affinitätsligandenträger, der eine poröse Festphase mit immobilisierten Affinitätsliganden und optional weitere Vorreaktionsreaktanten aufweist,

- wobei der Strömungswiderstand des Markierungsmittelträgers im Verhältnis zum Strömungswiderstand der stromab des Markierungsmittelträgers befindlichen Anordnung derart ist, dass ein erstes Teilvolumen der Probe den Markierungsmittelträger schneller durchströmt als ein stromab des Markierungsmittelträgers benachbart zu diesem befindliches poröses Durchströmelement, bei dem es sich entweder um den Affinitätsligandenträger oder ein Abstandselement zur Beabstandung des Affinitätsligandenträgers von dem Markierungsmittelträger handelt.

2. Vorrichtung nach Anspruch 1, **dadurch gekennzeichnet, dass** der Strömungswiderstand des Markierungsmittelträgers kleiner ist als der Strömungswiderstand des stromab des Markierungsmittelträgers befindlichen Durchströmelements, wobei die Strömungswiderstände des Markierungsmittelträgers und des Durchströmelements insbesondere durch deren Durchlässigkeitsbeiwerte definiert sind und dass der Durchlässigkeitsbeiwert des Markierungsmittelträgers größer ist als der Durchlässigkeitsbeiwert des Durchströmelements, wobei vorzugsweise der Durchlässigkeitsbeiwert des Markierungsmittelträgers etwa das 1,5 bis 2,5-fache des Durchlässigkeitsbeiwerts des Durchströmelements beträgt.

3. Vorrichtung nach Anspruch 1 oder 2, **dadurch gekennzeichnet, dass** der Markierungsmittelträger aus einer Fasern aufweisenden Festphase gebildet ist, wobei die Fasern im wesentlichen in Längserstreckung gerichtet sind und die Festphase insbesondere einen Porenanteil von 20 % bis 80 % mit einem mittleren Porendurchmesser von 5 $\mu$m bis 200 $\mu$m aufweist und die Fasern Polyolefin, Polyester, Zellulose, Zellulosederivat oder Glas aufweisen, und/oder dass der Affinitätsligandenträger ein gesintertes Substrat aus vorzugsweise Polymermaterial, wie z.B. Polyolefin oder Glas aufweist.

4. Vorrichtung nach einem der Ansprüche 1 bis 3, **dadurch gekennzeichnet, dass** das Gesamtvolumen der Probe mindesten gleich der Summe aus dem ersten Teilvolumen und einem zweiten Teilvolumen ist, das nach dem Füllen des Markierungsmittelträgers mit dem ersten Volumen der Probe zunächst in der Probenkammer verbleibt, wobei das zweite Teilvolumen mindestens gleich dem Gesamtporenvolumen sämtlicher von der Probe zum Zwecke des affinitätschromatographischen Tests zu durchströmender Elemente des Probengefäßes ist, und insbesondere mindestens gleich 110 % oder 120 % dieses Gesamtporenvolumens ist.

5. Vorrichtung nach einem der Ansprüche 1 bis 4, **dadurch gekennzeichnet, dass** zumindest das erste Teilvolumen der Probe während des Durchflusses durch das Probenanalysegefäßes mit Markierungsmittel und optional mit weiteren Vorreaktionsreaktanten reagiert oder sich mit Markierungsmittel reproduzierbar vermischt.

6. Vorrichtung nach einem der Ansprüche 1 bis 5, mit

- einem Gefäß, das ein oberes Ende mit einer Probeneinlassöffnung und ein unteres Ende mit einer Auslassöffnung aufweist,
- wobei das Gefäß von der Probeneinlassöffnung bis zur Auslassöffnung von einer zu analysierenden, wässrigen Probe durchströmbar ist und
- wobei das Gefäß versehen ist mit

- einer sich an die Probeneinlassöffnung anschließenden Probenkammer,
- mehreren Affinitätsligandenträgern, die jeweils eine poröse Festphase mit immobilisierten Affinitätsliganden aufweisen, und
- porösen Abstandselementen zur Beabstandung benachbarter Affinitätsligandenträger,

**dadurch gekennzeichnet,**

- **dass** der Strömungswiderstand der Abstandselemente verschieden ist von dem Strömungswiderstand der Affinitätsligandenträger.

7. Vorrichtung nach Anspruch 6, **dadurch gekennzeichnet, dass** mindestens ein Affinitätsligandenträger, vorzugsweise drei Affinitätsligandenträger mit zwei alternierend zu dem einen Affinitätsligandenträger, vorzugsweise vier alternierend zu dem Affinitätsligandenträger angeordneten Abstandselementen vorgesehen sind.

8. Vorrichtung nach Anspruch 7, **dadurch gekennzeichnet, dass** die Strömungswiderstände der Abstandselemente und der Affinitätsligandenträger durch deren Durchlässigkeitsbeiwerte definiert sind und dass der Durchlässigkeitsbeiwert der Abstand-

17 **EP 2 264 461 A1** 18

selemente niedriger oder höher ist als der Durchlässigkeitsbeiwert der Affinitätsligandenträger, wobei insbesondere der Durchlässigkeitsbeiwert der Affinitätsligandenträger etwa das 1,5 bis 2,5-fache vorzugsweise das 0,5 bis 0,75-fache des Durchlässigkeitsbeiwerts der Abstandselemente beträgt.

9. Vorrichtung nach einem der Ansprüche 6 bis 8, **dadurch gekennzeichnet, dass** die Abstandselemente aus Sintermaterial bestehen.

10. Vorrichtung nach einem der Ansprüche 1 bis 9, **dadurch gekennzeichnet, dass** ein Abfallbehältnis vorgesehen ist, das unterhalb der Auslassöffnung des Probenanalysegefäßes angeordnet ist oder dass die Auslassöffnung des Probenanalysegefäßes mit einem sich bei Benetzung mit Flüssigkeit selbsttätig verschließenden Verschlusselement versehen ist.

11. Vorrichtung nach einem der Ansprüche 1 bis 10, **dadurch gekennzeichnet, dass** die Affinitätsliganden ausgewählt sind aus Stoffen, die umfassen: Proteine wie Antikörper, Protein A, Protein G, Streptavidin, native oder rekombinante Oberflächenantigene von Mikroorganismen, somatischen Zellen, oder Viren, Lektinen oder Nukleinsäuren wie einsträngige RNA, DNA Sonden, aptamere oder oligomeren Verbindungen wie Peptiden, Oligosacheriden oder niedermolekularen Verbindungen wie Haptenen, Zuckern.

12. Vorrichtung nach einem der Ansprüche 1 bis 11, **dadurch gekennzeichnet, dass** das Markierungsmittel hergestellt wird durch Konjugation eines Proteins, einer Nukleinsäure, eines Oligopeptids, oder eines Haptens mit einem Markermolekül, das optisch (insbesondere photometrisch, fluorimetrisch, luminometrisch oder phosphorimetrisch) oder magnetisch oder elektrochemisch (insbesondere amperometrisch, potentiometrisch, konduktionmetrisch) detektierbar ist.

13. Vorrichtung nach einem der Ansprüche 1 bis 12, **dadurch gekennzeichnet, dass** die Strömungswiderstände bzw. Durchlässigkeitsbeiwerte des Markierungsmittelträgers, des bzw. der Affinitätsligandenträger und des oder der Abstandselemente insbesondere durch Wahl der Porengröße und/oder die Benetzungseigenschaften bzw. Grenzflächenspannung, d.h. die Wechselwirkungsenergie der Oberflächen der Festphasen zur Flüssigphase vorgebbar sind.

14. Verwendung einer Vorrichtung nach einem der vorhergehenden Ansprüche zur Durchführung eines affinitätschromatographischen Probentests, wobei

   - die Menge an zu analysierender, wässriger Probe derart gewählt wird, dass Markierungsmittel und/oder Markierungsmittel-Analyt-Komplexe, das/die in dem bzw. mindestens einem der Affinitätsligandenträger unspezifisch gebunden ist/sind, von nachlaufender Probe aus dem betreffenden Affinitätsligandenträger herausgewaschen wird/werden.

**Fig.1**

**Fig.2**

**Fig.3**

**Fig.4**

**Fig.5**

**Fig.6**

**Fig.7**

**Fig.8**

**Fig.9**

**Fig.10**

**Fig.11**

**Fig.12**

**Fig.13**

**Fig.14**

Europäisches
Patentamt

European
Patent Office

Office européen
des brevets

**EUROPÄISCHER RECHERCHENBERICHT**

Nummer der Anmeldung

EP 09 16 3263

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | DE 195 00 862 A1 (ABION OHG [DE]) 20. Juli 1995 (1995-07-20) *Seite 3, Zeile 7 - Seite 5, Zeile 47, Abbildung 1* ----- | 1-14 | INV. G01N33/543 B01L3/00 G01N30/60 |
| Y | DE 10 2007 025311 A1 (SENOVA GES FUER BIOWISSENSCHAF [DE]) 4. Dezember 2008 (2008-12-04) *Absatz [0008]-Absatz [0024], Absatz [0039]-Absatz [0042]* ----- | 1-14 | |
| Y | WO 2005/124347 A (ANI BIOTECH OY [FI]; SARAMAEKI MIKA [FI]; NISKANEN AIMO [FI]) 29. Dezember 2005 (2005-12-29) *Seite 3, Zeile 18 - Seite 5, Zeile 18, Seite 7, Zeile 27 - Seite 9, Zeile 12* ----- | 1-14 | |
| Y | WO 02/08727 A (EY LAB INC [US]) 31. Januar 2002 (2002-01-31) *Seite 4, Zeile 23 - Seite 7, Zeile 26* ----- | 1-14 | RECHERCHIERTE SACHGEBIETE (IPC) G01N B01L |

Der vorliegende Recherchenbericht wurde für alle Patentansprüche erstellt

| Recherchenort | Abschlußdatum der Recherche | Prüfer |
|---|---|---|
| München | 11. September 2009 | Lindberg, Pia |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument
..........................................................................................
& : Mitglied der gleichen Patentfamilie, übereinstimmendes
   Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 09 16 3263

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-09-2009

| Im Recherchenbericht angeführtes Patentdokument | Datum der Veröffentlichung | Mitglied(er) der Patentfamilie | | Datum der Veröffentlichung |
|---|---|---|---|---|
| DE 19500862 A1 | 20-07-1995 | AU | 5882994 A | 01-08-1995 |
| | | WO | 9519569 A1 | 20-07-1995 |
| | | EP | 0739487 A1 | 30-10-1996 |
| | | JP | 9507577 T | 29-07-1997 |
| DE 102007025311 A1 | 04-12-2008 | WO | 2008145722 A1 | 04-12-2008 |
| WO 2005124347 A | 29-12-2005 | CA | 2570383 A1 | 29-12-2005 |
| | | EP | 1782067 A1 | 09-05-2007 |
| WO 0208727 A | 31-01-2002 | AU | 2456402 A | 05-02-2002 |
| | | CA | 2416886 A1 | 31-01-2002 |
| | | CN | 1447913 A | 08-10-2003 |
| | | EP | 1320739 A1 | 25-06-2003 |
| | | JP | 2004508541 T | 18-03-2004 |
| | | US | 6632681 B1 | 14-10-2003 |

EPO FORM P0461

Für nähere Einzelheiten zu diesem Anhang : siehe Amtsblatt des Europäischen Patentamts, Nr.12/82

# EP 2 264 461 A1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 0306772 B
- EP 0634015 B
- EP 0557288 B
- WO 9519569 A
- DE 102004006470
- DE 10309132
- WO 200845722 A